(19) Europäisches Patentamt
European Patent Office
Office européen des brevets

(11) EP 2 045 234 A1

(12) **EUROPEAN PATENT APPLICATION**
published in accordance with Art. 153(4) EPC

(43) Date of publication:
**08.04.2009 Bulletin 2009/15**

(21) Application number: **07763975.5**

(22) Date of filing: **02.07.2007**

(51) Int Cl.:
***C07C 215/28*** *(2006.01)* ***C07C 213/10*** *(2006.01)*
***A61K 31/137*** *(2006.01)* ***A61P 11/06*** *(2006.01)*
***A61P 11/08*** *(2006.01)*

(86) International application number:
**PCT/CN2007/002060**

(87) International publication number:
**WO 2008/006295 (17.01.2008 Gazette 2008/03)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **07.07.2006 CN 200610090242**

(71) Applicant: **Shenyang Pharmaceutical University**
**Shenyang,**
**Liaoning 110016 (CN)**

(72) Inventors:
- **CHENG, Maosheng**
  **Liaoning 110016 (CN)**
- **PAN, Li**
  **Liaoning 110016 (CN)**
- **WANG, Dongkai**
  **Liaoning 110016 (CN)**
- **XING, Ruijuan**
  **Liaoning 110016 (CN)**
- **CHEN, Xuyao**
  **Liaoning 110016 (CN)**
- **ZHAO, Dongmei**
  **Liaoning 110016 (CN)**

(74) Representative: **Weller, Wolfgang et al**
**Witte, Weller & Partner**
**Patentanwälte**
**Postfach 10 54 62**
**70047 Stuttgart (DE)**

(54) **NEW OPTICAL ACTIVE PHENYLETHANOL AMINES AND PREPARING METHOD THEREOF**

(57) The invention provides compounds of formula (I) having (-) or (+) configuration, or pharmaceutically acceptable salts thereof, wherein $R_1$ is H or halo; $R_2$ is $CF_3$, CN, or halo; $R_3$ is linear or branched alkyl having 1 to 6 carbon atoms, or cycloalkyl having 3 to 6 carbon atoms. The invention also relates to methods for preparing the said compounds and the composition comprising the same. The compounds of the present invention have the effect of $\beta_2$- receptor agonist and can be used for the treatment of asthma or bronchitis.

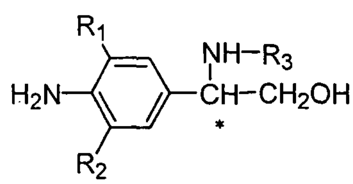

(I)

EP 2 045 234 A1

## Description

### Technical Field

The present invention relates to new optically active phenylethanolamines compounds having the effect of $\beta_2$-receptor agonist, which are in particular useful for the treatment of asthma or bronchitis. The invention also relates to methods for preparing the said compounds, the pharmaceutical composition comprising the same and the use thereof.

### Background of the invention

The asthma and bronchitis are common diseases. In most case, antibiotics are used in the treatment of asthma and bronchitis, which are not very effective and have some side effect in long-term usage. $\beta_2$-Receptor agonists are well known as anti-asthma agents. However, these agents are still deficient in effects and physical and chemical properties.

Chinese Patent No. 01128234.7 discloses a new phenylethanolamines compounds having a good effect of $\beta_2$-receptor agonist. However, no teaching is given in the patent for any optically active isomers of the phenylethanolamines compounds.

### Disclosure of the invention

An objective of the invention is to provide a new optically active phenylethanolamines compounds, which show a higher $\beta_2$-receptor agonist activity and a lower toxicity than their racemic mixture.

The invention provides compounds of formula (I) having (-) or (+) configuration or pharmaceutically acceptable salts thereof

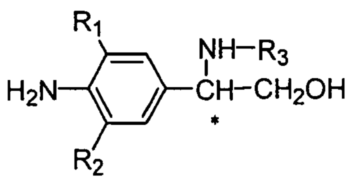

(I)

wherein

$R_1$ is H or halo; $R_2$ is $CF_3$, CN, or halo; $R_3$ is linear or branched alkyl having 1 to 6 carbon atoms, or cycloalkyl having 3 to 6 carbon atoms,

or pharmaceutically acceptable salts thereof.

Preferably, in formula I, $R_1$ is Cl or Br; $R_2$ is $CF_3$, CN, or F; and $R_3$ is linear or branched alkyl having 3 to 6 carbon atoms.

In a preferred embodiment according to the invention, the compounds of formula (I) have (-) configuration.

More preferably, the compounds of the invention are selected from the group consisting of:

(-)-2-(3-chloro-4-amino-5-trifluoromethylphenyl)-2-tert-butylamino-ethanol hydrochloride,
(+)-2-(3-chloro-4-amino-5-trifluoromethylphenyl)-2-tert-butylamino-ethanol hydrochloride,
(-)-2-(3-trifluoromethyl-4-aminophenyl)-2-tert-butylamino-ethanol hydrochloride,
(+)-2-(3-trifluoromethyl-4-aminophenyl)-2-tert-butylamino-ethanol hydrochloride,
(-)-2-(3-chloro-4-amino-5-cyanophenyl)-2-tert-butylamino-ethanol hydrochloride,
(+)-2-(3-chloro-4-amino-5-cyanophenyl)-2-tert-butylamino-ethanol hydrochloride,
(-)-2-(3-bromo-4-amino-5-cyanophenyl)-2-tert-butylamino-ethanol hydrochloride, and
(+)-2-(3-bromo-4-amino-S-cyanophenyl)-2-tert-butylamino-ethanol hydrochloride.

The term "pharmaceutically acceptable salt" used herein refers to conventional acid-addition salts which retain the biological effectiveness and properties of the compounds of formula I and which are formed from suitable non-toxic organic or inorganic acids. Examples of acid-addition salts include those derived from inorganic acids such as hydrochloric acid, hydrobromic acid, hydroiodic acid, sulfuric acid, phosphoric acid and nitric acid, those derived from organic acids such as acetic acid, tartaric acid, salicylic acid, methanelsulfonic acid, butanedioic acid, citric acid, malic acid, lactic acid, fumaric acid, and the like. In particular, the pharmaceutically acceptable salts of the compounds of formula I are preferably hydrochloride or hydrobromide.

Both above optically active isomers having (+) and (-) configuration respectively possess $\beta_2$-receptor agonist activity, wherein the isomer having (-) configuration possess a $\beta_2$-receptor agonist activity 3 to 7 folds higher than that of the isomer having (+) configuration and 2 to 5 folds higher than that of the racemic mixture. Furthermore, the toxicity of the isomer having (-) configuration is less than those of racemic mixture and the isomer having (+) configuration. The invention further provides a method for preparing the compounds of formula (I) through resolution, comprising reacting the racemic mixture of formula (II)

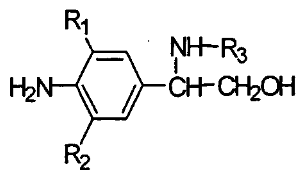 (II)

wherein $R_1$, $R_2$ and $R_3$ are as defined above,

With a compound selected from the group consisting of: D(-)-tartaric acid, L(+)-tartaric acid, dibenzoyl-D-tartaric acid, dibenzoyl-L-tartaric acid, (+)(-)camphor-10-sulfonic acid, L(-)-malic acid, L(+)-mandelic acid, d-α-bromo-camphorsulfonic acid and 1-quininic acid, under an anhydrous condition to form a salt; and crystallizing the salt for two or more times to resolve it into compounds of formula (I) of Claim 1 having optical activity.

Preferably, crystallization is performed for 2 to 5 times in above resolution. More preferably, the crystallization is conducted for two or three times.

In above resolution, the reaction solvent is preferably an alcohol (for example, absolute ethyl alcohol), an ether (for example, absolute ethyl ether) or a hydrocarbon (for example, petroleum ether). The reaction is preferably performed at a temperature between room temperature and reflux temperature. The produce yield is typically 50 to 60%.

The present invention also provides a pharmaceutical composition comprising the compounds of formula (I) having (+) or (-) configuration and pharmaceutically acceptable excipients.

The "pharmaceutically acceptable excipients" means an excipient that is useful in pharmaceutical fields that is generally safe, non-toxic and not biologically or otherwise undesirable. These excipients include lactose, starch, water, alcohol, and the like.

The pharmaceutical composition according to the present invention can also include propellents, antiseptics, solubilizing agents, stabilizing agents, moistening agents, emulsifiers, sweetening agents, colorants, flavoring agents, salts for adjusting osmotic pressure, buffer, coating agents, antioxidants, and the like. The pharmaceutical composition according to the present invention can also comprise other therapeutically valuable substances, for example other active ingredients other than the compound of formula I.

The pharmaceutical composition according to the present invention can be formulated into tablets, capsules, solutions, sprays, injections, and the like. It can be administrated by oral, parenteral, spraying, inhaling through oral or nasal cavity or other forms.

The compounds of the present invention have the effect of $\beta_2$-receptor agonist and can be used for the treatment of asthma and bronchitis. Accordingly, the present invention further relates to use of the compounds of formula (I) having (+) or (-) configuration in the preparation of medicaments having effect of $\beta_2$-preceptor agonist. The present invention also relates to use of the compound of formula (I) having (+) or (-) configuration in the preparation of medicaments for the treatment of asthma and bronchitis.

The compounds of the present invention can be administrated in a therapeutically effective amount. The "a therapeutically effective amount" means an amount that effectively prevent, alleviate, improve the diseases conditions. The "a therapeutically effective amount" can be determined by those skilled in the art.

The therapeutically effective amount or dose may be changed in a broad scope, and may be adjusted according to requirement of individual case. Typically, for adults with about 70Kg weight, preferably the dose is about 10 μg-20 mg/day, more preferably 50 μg-10 mg/day when administrated in oral or parenteral form. When required, the upper limit and low limit of dose can be exceeded. The daily dose can be administrated alone or divided in several times.

The following examples illustrate synthesis method of these compounds.

EXAMPLE 1

(-)-2-(3-chloro-4-amino-5-trifluoromethylphenyl)-2-tert-butylamino-ethanol hydrochloride and
(+)-2-(3-chloro-4-amino-5-trifluoromethylphenyl)-2-tert-butylamino-ethanol hydrochloride

a) (-)-2-(3-chloro-4-amino-5-trifluoromethylphenyl)-2-tert-butylamino-ethanol hydrochloride

4.45 g (0.0143 mol) of 2-(3-chloro-4-amino-5-trifluoromethylphenyl)-2-tert-butylamino-ethanol was dissolved in 53.4 ml of absolute ethanol. A solution of 2.57g (0.00717mol)) dibenzoyl-D-tartaric acid in 25.7 ml absolute ethanol was dropwise added and 188 ml of petroleum ether (bp. 60~90˚C) was then added. After mixing for 1 hour, the mixture was filtered and dried to obtain 2-(3-chloro-4-amino-5-trifluoromethylphenyl) -2-tert-butylamino-ethanol dibenzoyl-D-tartrate (2.9 g). Yield: 82.6%.

2.9 g of 2-(3-chloro-4-amino-5-trifluoromethylphenyl)-2-tert-butylamino-ethanol dibenzoyl-D-tartrate was added to 60 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 1.7 g of (-)-2-(3-chloro-4-amino-5-trifluoromethylphenyl)-2-tert-butylamino-ethanol was obtained. ee% = 92.2%. Yield = 76.4%.

The second resolution was carried out with dibenzoyl-D-tartaric acid and the procedures were same as above. The obtained ether solution was added with HCl solution in isopropanol to reach pH = 2. After filtration and drying, 1.43 g of (-)-2-(3-chloro-4-amino-5-trifluoromethylphenyl)-2-tert-butylamino-ethanol hydrochloride was obtained. ee% = 99.0%, mp: 209~210.7 ˚C (dec.). Overall yield: 57.4%.

$[\alpha]_D$ = -18.8˚ (c=0.5; absolute methanol)

b) (+)-2-(3-chloro-4-amino-5-trifluoromethylphenyl)-2-tert-butylamino-ethanol hydrochloride

4.45 g (0.0143 mol) of 2-(3-chloro-4-amino-5-trifluoromethylphenyl)-2-tert-butylamino-ethanol was dissolved in 53.4 ml of absolute ethanol. A solution of 2.57g (0.00717mol) dibenzoyl-L-tartaric acid in 25.7 ml absolute ethanol was dropwise added and 188 ml of petroleum ether (bp. 60-90˚C) was then added. After mixing for 1 hour, the mixture was filtered and dried to obtain 2-(3-chloro-4-amino-5-trifluoromethylphenyl) -2-tert-butylamino-ethanol dibenzoyl-L-tartrate (3.2 g). Yield: 91.2%.

'3.2 g of 2-(3-chloro-4-amino-5-trifluoromethylphenyl)-2-tert-butylamino-ethanol dibenzoyl-L-tartrate was added to 65 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 1.9 g of (+)-2-(3-chloro-4-amino-5-trifluoromethylphenyl) -2-tert-butylamino-ethanol was obtained. ee% = 94.4%. Yield = 85.4%.

The second resolution was carried out with dibenzoyl-L-tartaric acid and the procedures were same as above. The obtained ether solution was added with HCl solution in isopropanol to reach pH = 2. After filtration and drying, 1.52 g of (+)-2-(3-chloro-4-amino-5-trifluoromethylphenyl)-2-tert-butylamino-ethanol hydrochloride was obtained. ee% = 99.0%, mp: 209.6~211.0 ˚C (dec.). Overall yield: 61.0%.

$[\alpha]_D$ = +18.5˚ (c=0.5; absolute methanol)

EXAMPLE 2

(-)-2-(3-trifluoromethyl-4-aminophenyl)-2-tert-butylamino-ethanol hydrochloride, and
(+)-2-(3-trifluoromethyl-4-aminophenyl)-2-tert-butylamino-ethanol hydrochloride

a) (-)-2-(3-trifluoromethyl-4-aminophenyl)-2-tert-butylamino-ethanol hydrochloride

4.5 g (0.0163 mol) of 2-(3-trifluoromethyl-4-aminophenyl)-2-tert-butylamino-ethanol was dissolved in 65 ml of isopropanol. A solution of 1.23 g (0.0082 mol) L-tartaric acid in 25 ml isopropanol was then dropwise added. After mixing for 2 hour, the mixture was filtered and dried to obtain 2-(3-trifluoromethyl-4-aminophenyl)-2-tert-butylamino-ethanol L-tartrate (2.24 g). Yield: 78.1%.

2.24 g of 2-(3-trifluoromethyl-4-aminophenyl)-2-tert-butylamino-ethanol L-tartrate was added to 50 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 1.67 g of (-)-2-(3-trifluoromethyl-4-aminophenyl)-2-tert-butylamino-ethanol was obtained. ee% = 90.0%. Yield = 74.2%.

The second resolution was carried out with L-tartaric acid and the procedures were same as above. The obtained ether solution was added with HCl solution in isopropanol to reach pH = 2. After filtration and drying, 1.38 g of (-)-2-(3-trifluoromethyl-4-aminophenyl)-2-tert-butylamino-ethanol hydrochloride was obtained. ee% = 95.0%. Overall yield: 54.0%.

b) (+)-2-(3-trifluoromethyl-4-aminophenyl)-2-tert-butylamino-ethanol hydrochloride

4.5 g (0.0163 mol) of 2-(3-trifluoromethyl-4-aminophenyl)-2-tert-butylamino -ethanol was dissolved in 65 ml of

isopropanol. A solution of 1.23 g (0.0082 mol) D-tartaric acid in 25 ml isopropanol was then dropwise added. After mixing for 2 hour, the mixture was filtered and dried to obtain 2-(3-trifluoromethyl-4-aminophenyl) -2-tert-butylamino-ethanol D-tartrate (2.3 g). Yield: 80.2%.

2.3 g of 2-(3-trifluoromethyl-4-aminophenyl)-2-tert-butylamino-ethanol D-tartrate was added to 52 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 1.63 g of (+)-2-(3-trifluoromethyl-4-aminophenyl)-2-tert-butylamino-ethanol was obtained. ee% = 90.6%. Yield = 72.4%.

The second resolution was carried out with D-tartaric acid and the procedures were same as above. The obtained ether solution was added with HCl solution in isopropanol to reach pH = 2. After filtration and drying, 1.31 g of (+)-2-(3-trifluoromethyl-4-aminophenyl)-2-tert-butylamino-ethanol hydrochloride was obtained. ee% = 94.6%. Overall yield: 51.5%.

EXAMPLE 3

(-)-2-(3-chloro-4-amino-5-cyanophenyl)-2-tert-butylamino-ethanol hydrochloride, and
d-2-(3-chloro-4-amino-5-cyanophenyl)-2-tert-butylamino-ethanol hydrochloride

a) (-)-2-(3-chloro-4-amino-5-cyanophenyl)-2-tert-butylamino-ethanol hydrochloride

2.5 g (0.0093 mol) of 2-(3-chloro-4-amino-5-cyanophenyl)-2-tert-butylamino -ethanol was dissolved in 50 ml of anhydrous ethanol. A solution of 1.67 g (0.0047 mol) dibenzoyl-D-tartaric acid in 16.7 ml anhydrous ethanol was dropwise added. 200 ml of anhydrous ethyl ether was then added. After mixing for 1 hour, the mixture was filtered and dried to obtain 2-(3-chloro-4-amino-5-cyanophenyl)-2-tert-butylamino-ethanol dibenzoyl-D-tartrate (1.58 g). Yield: 75.6%.

1.58 g of 2-(3-chloro-4-amino-5-cyanophenyl)-2-tert-butylamino-ethanol dibenzoyl -D-tartrate was added to 32 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 0.9 g of (-)-2-(3-chloro-4-amino-5-cyanophenyl)-2-tert- butylamino-ethanol was obtained. ee% = 89.7%. Yield = 72.0%.

The second resolution was carried out with dibenzoyl-D-tartaric acid and the procedures were same as above. The obtained ether solution was added with HCl solution in isopropanol to reach pH = 2. After filtration and drying, 0.77 g of (-)-2-(3-chloro-4-amino-5-cyanophenyl)-2-tert-butylamino-ethanol hydrochloride was obtained. ere% = 97.0%. Overall yield: 54.4%.

$[\alpha]_D$ = -19.3° (c=0.5; absolute methanol)

b) (+)-2-(3-chloro-4-amino-5-cyanophenyl)-2-tert-butylamino-ethanol hydrochloride

2.5 g (0.0093 mol) of 2-(3-chloro-4-amino-5-cyanophenyl)-2-tert-butylamino -ethanol was dissolved in 50 ml of anhydrous ethanol. A solution of 1.67 g (0.0047 mol) dibenzoyl-L-tartaric acid in 16.7 ml anhydrous ethanol was dropwise added. 200 ml of anhydrous ethyl ether was then added. After mixing for 1 hour, the mixture was filtered and dried to obtain 2-(3-chloro-4-amino-5-cyanophenyl)-2-tert-butylamino -ethanol dibenzoyl-L-tartrate (1.67 g). Yield: 80.2%.

1.67 g of 2-(3-chloro-4-amino-5-cyanophenyl)-2-tert-butylamino-ethanol dibenzoyl -L-tartrate was added to 33 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 0.94 g of (+)-2-(3-chloro-4-amino-5-cyanophenyl)-2-tert-butylamino-ethanol was obtained. ee% = 89.4%. Yield = 75.9%.

The second resolution was carried out with dibenzoyl-L-tartaric acid and the procedures were same as above. The obtained ether solution was added with HCl solution in isopropanol to reach pH = 2. After filtration and drying, 0.82 g of (+)-2-(3-chloro -4-amino-5-cyanophenyl)-2-tert-butylamino-ethanol hydrochloride was obtained. ee% = 96.0%. Overall yield: 58.0%.

$[\alpha]_D$ = +18.5° (c=0.5; absolute methanol)

EXAMPLE 4

(-)-2-(3-bromo-4-amino-5-cyanophenyl)-2-tert-butylamino-ethanol hydrochloride, and

(+)-2-(3-bromo-4-amino-5-cyanophenyl)-2-tert-butylamino-ethanol hydrochloride

a) (-)-2-(3-bromo-4-amino-5-cyanophenyl)-2-tert-butylamino-ethanol hydrochloride

2.5 g (0.008 mol) of 2-(3-bromo-4-amino-5-cyanophenyl)-2-tert-butylamino -ethanol was dissolved in 50 ml of anhydrous ethanol. A solution of 1.43 g (0.004 mol) dibenzoyl-L-tartaric acid in 14.3 ml anhydrous ethanol was dropwise added. 193 ml of anhydrous ethyl ether was then added. After mixing for 1 hour, the mixture was filtered and dried to obtain 2-(3-bromo-4-amino-5-cyanophenyl)-2-tert-butylamino-ethanol dibenzoyl-L-tartrate (1.61 g). Yield: 82.0%.

1.61 g of 2-(3-bromo-4-amino-5-cyanophenyl)-2-tert-butylamino-ethanol dibenzoyl -L-tartrate was added to 32 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 0.96 g of (-)-2-(3-bromo-4-amino-5-cyanophenyl)-2-tert-butylamino-ethanol was obtained. ee% = 90.2%. Yield = 76.6%.

The second resolution was carried out with dibenzoyl-L-tartaric acid and the procedures were same as above. The obtained ether solution was added with HCl solution in isopropanol to reach pH = 2. After filtration and drying, 0.75 g of (-)-2-(3-bromo-4-amino-5-cyanophenyl)-2-tert-butylamino-ethanol hydrochloride was obtained. ere% = 95.0%. Overall yield: 54.0%.

b) (+)-2-(3-bromo-4-amino-5-cyanophenyl)-2-tert-butylamino-ethanol hydrochloride

2.5 g (0.008 mol) of 2-(3-bromo-4-amino-5-cyanophenyl)-2-tert-butylamino -ethanol was dissolved in 50 ml of anhydrous ethanol. A solution of 1.43 g (0.004 mol) dibenzoyl-D-tartaric acid in 25.7 ml anhydrous ethanol was dropwise added. 193 ml of anhydrous ethyl ether was then added. After mixing for 1 hour, the mixture was filtered and dried to obtain 2-(3-bromo-4-amino-5-cyanophenyl)-2-tert-butylamino-ethanol dibenzoyl-D-tartrate (1.59 g). Yield: 81.0%.

1.59 g of 2-(3-bromo-4-amino-5-cyanophenyl)-2-tert-butylamino-ethanol dibenzoyl -D-tartrate was added to 32 ml water. After mixing, 20% NaOH solution was added to adjust the mixture to pH = 10. The mixture was extracted with ethyl ether and dried over anhydrous sodium sulfate. After filtration, the ethyl ether was removed under reduced pressure and 0.97 g of (+)-2-(3-bromo-4-amino-5-cyanophenyl)-2-tert-butylamino -ethanol was obtained. ee% = 89.4%. Yield = 77.8%.

The second resolution was carried out with dibenzoyl-D-tartaric acid and the procedures were same as above. The obtained ether solution was added with HCl solution in isopropanol to reach pH = 2. After filtration and drying, 0.78 g of (+)-2-(3-bromo -4-amino-5-cyanophenyl)-2-tert-butylamino-ethanol hydrochloride was obtained. eye% = 95.5%. Overall yield: 56.0%.

EXAMPLE 5 General Tablets

1) Formulation of the preparation

| | |
|---|---|
| The compound of the invention | 0.05 g |
| Lactose | 82.45 g |
| Starch | 33.00 g |
| PVP | 4.00 g |
| Magnesium stearate | 0.50 g |
| Preparing into | 1000 tablets |

2) Preparation process

Above amount of compound of the invention was weighed and dissolved in a suitable amount of ethanol together with PVP. Lactose and starch were mixed and added to the solution to be wetted homogeneously. Wet particles were made by using a 1.5 mm sieve mesh and dried by aeration drying at 50˚C. A 1.0 mm sieve was used for granulation. The granulate was mixed homogeneously with magnesium stearate and compressed. 120 mg/Tablet. A flat punch of 7 mm was used.

EXAMPLE 6 Film coated tablets

1) Formulation of the preparation

| | |
|---|---|
| The compound of the invention | 0.055 g |
| Lactose | 82.475 g |
| Potato starch | 33.00 g |
| PVP | 4.00 g |
| Magnesium stearate | 0.50 g |
| Preparing into | 1000 tablets |

2) Preparation process

Above amount of compound of the invention was weighed and dissolved in a suitable amount of ethanol together with PVP. Lactose and starch were mixed and added to the solution to be wetted homogeneously. Wet particles were made by using a 1.5 mm sieve mesh and dried by aeration drying at 50˚C. A 1.0 mm sieve was used for granulation. The granulate was mixed homogeneously with magnesium stearate and compressed. 120 mg/Tablet. A deeply dented punch of 7 mm was used. The tablet was coated with sugar film. 200 mg/film coated tablet.

EXAMPLE 7 Capsules

1) Formulation of the preparation

| | |
|---|---|
| The compound of the invention | 0.025 g |
| Lactose | 59.975 g |
| Corn starch | 60 g |
| Preparing into | 1000 capsules |

2) Preparation process

Above amount of compound of the invention was weighed, mixed homogeneously with lactose and starch, and filled into capsules. The contents of each capsule weighed 120 mg.

EXAMPLE 8 Injection solutions

1) Formulation of the preparation

| | |
|---|---|
| The compound of the invention | 0.02 g |
| Citric acid | 2.5 g |
| $Na_2HPO_4$ | 7.5 g |
| NaCl | 4.6 g |
| Water for injection | q.s. ad 2000 ml |
| Preparing into | 1000 vials |

2) Preparation process

Above amount of compound of the invention was weighed and added to 1000 ml of water for injection. Above

amounts of citric acid, $Na_2HPO_4$ and NaCl were then added to the solution under stirring and dissolve completely. Active carbon was added and the solution was agitated at 80˚C for 20 min. The active carbon was then removed by filtration through 0.2 μm microporous membrane. Another 1000 ml of water for injection was added. The content of the compound and the pH of the solution were measured. The solution was then encapsulated into 2 ml ampoules and sterilized at 120˚C for 20 min.

EXAMPLE 9 Suppository

1) Formulation of the preparation

| | |
|---|---|
| The compound of the invention | 0.050 g |
| Suppository substrate | 1699.95 g |
| Preparing into | 1000 pieces |

2) Preparation process

Above amount of the compound of the invention was melt with substrate at 40˚C and filled into mould. After cooling to a temperature below 37˚C, the suppository was obtained.

EXAMPLE 10 Syrups

1) Formulation of the preparation

| | |
|---|---|
| The compound of the invention | 0.0005 g |
| Benzoic acid | 0.1 g |
| Malic acid | 1.0 g |
| Sucrose | 50.0 g |
| Flavoring orange essence | 1.0 g |
| Red pigment | 0.05 g |
| Distilled water | q.s. ad 100.0 mL |

2) Preparation process

60 ml distilled water was heated to 80˚C and above amounts of benzoic acid, malic acid, the compound of the invention, red pigment and sucrose were dispensed therein sufficiently. Flavoring orange essence was added and water was added to full amount. The syrup was obtained after filtration.

EXAMPLE 11 Powders

1) Formulation of the preparation

| | |
|---|---|
| The compound of the invention | 0.05 g |
| Lactose | 0.25 g |
| Filled into capsules of size 3 | 1000 capsules |

2) Preparation process

Above amount of the compound of the invention was weighed and placed in a fluidized bed and pulverized with a supersonic airflow to obtain ultramicro dry powder with a particle size from 1 μm to 5 μm. A lactose excipient with

a maximum particle size of below 200 μm was produced through high speed grinding and crushing. The ultramicro dry powder and the lactose excipient were mixed homogeneously via method of "increasing by equal amounts" and filled into capsules of size 3.

EXMAPLE 12 Inhalants

1) Formulation of the preparation

| | |
|---|---|
| The compound of the invention | 0.05 g |
| Hydroxypropyl methylcellulose | 0.3 g |
| Microcrystalline cellulose | 1.5 g |
| Lactose | 9 g |
| Preparing into | 1000 vesicles |

2) Preparation process

The compound of the invention, hydroxypropyl methylcellulose, microcrystalline cellulose and lactose were pulverized respectively and sieved through 200 mesh. 0.1 g of hydroxypropyl methylcellulose and 50%(v/v) ethanol/water solution were formulated into a 1%(w/v) hydroxypropyl methylcellulose ethanol/water solution. Above amount of sieved compound of the invention was dissolved in 8 ml of hydroxypropyl methylcellulose ethanol/water solution. 0.2 g of the sieved hydroxypropyl methylcellulose, 1.5 g of the sieved microcrystalline cellulose and 9 g of the sieved lactose were mixed homogeneously and then prepared into a soft material with previous hydroxypropyl methylcellulose ethanol/water solution containing the compound of the invention. The soft material was granulated with a sieve (30 mesh), dried at 60˚C in oven, and then granulated with two sieves (100 mesh and 400 mesh, respectively). The granulates between 100 mesh and 400 mesh were selected and filled into vesicles to obtain a vesicle-type dry powder inhalant.

EXAMPLE 13 Cataplasma

1) Formulation of the preparation

| | |
|---|---|
| The compound of the invention | 0.0025 g |
| Sodium carboxymethyl cellulose | 0.01 g |
| Sodium polyacrylate (Carborit 7S) | 0.025 g |
| Kaolin | 0.02 g |
| Glycerol | 0.5 ml |
| $AlCl_3$ | 0.0015 g |
| Distilled water | 4 ml |
| Laurocapram | 0.02 g |
| Citric acid (10%) | 0.2 ml |
| Oleic acid | 0.0015 g |
| Gelatin | 0.0075 g |
| Preparing into | 50 pieces |

2) Preparation process

Above amount of gelatin was weighed and swollen with water sufficiently. 60˚C water bath was utilized to facilitate the dissolution. Sodium carboxymethyl cellulose was added to the gelatin solution under stirring to obtain the first solution. Above amounts of sodium polyacrylate, $AlCl_3$, Laurocapram, kaolin, citric acid and the compound of the invention were mixed homogenously to obtain the second solution. The first solution was then mixed with the second solution and glycerol and oleic acid were added in indicated amounts. After mixing thoroughly, the mixture was plated

onto a non-woven fabric (250 x 300 $cm^2$), which was then covered with antisticking layers (5 cm x 6 cm) to produce 50 pieces of cataplasma.

EXAMPLE 14 Patches

1) Formulation

| | |
|---|---|
| Block copolymer of styrene-isoprene-styrene | 40.0 g |
| Terpene resin | 34.5 g |
| Aliphatic hydrocarbon resin | 10.0 g |
| The compound of the invention | 2.0 g (20% aqueous solution) |
| N-methyl-2-pyrrolidone | 5.0 g |
| α-monoisostearyl glycerol ether | 2.5 g |
| Isopropyl myristate | 5.0 g |
| Sorbitan fatty acid esters mixture | 1.0 g |
| Ethyl acetate | q.s. |

2) Preparation process

To a mixture of 40.0 g block copolymer of styrene-isoprene-styrene, 34.5 g terpene resin and 10.0 g aliphatic hydrocarbon resin, 2.0 g of the compound of the invention in form of 20% aqueous solution, 5.0 g N-methyl-2-pyrrolidone, 2.5 g α-monoisostearyl glycerol ether, 5.0 g isopropyl myristate, and 1.0 g sorbitan fatty acid esters mixture were added. A suitable amount of ethyl acetate was added and mixed homogeneously to form a plaster. The plaster was coated uniformly onto peelable films and dried with warm air. Supporting films were then adhered to the peelable films to press with rotating to manufacture adhesive patches.

EXAMPLE 15 Study of the effect of compounds of the invention on $\beta_2$-receptor

**Experimental Animals**

Guinea pigs (Hartley, purchased from the Experimental Animal Center of Shenyang Pharmaceutical University. Certificate of approval: SCXK (Liao) - 2003-011) of either sex weighting 400-500 g were used.

Reagent

Sample: All tested compounds were formulated into solution of $10^{-6}$ M.
Histamine phosphate solution: Shanghai LiZhu Biotechnology Co., Ltd. Lot No. 1703
Isoprenaline hydrochloride, ISO: Shanghai Harvest Pharmaceutical Co., Ltd. Lot No. 20040901

**Instruments**

S-501-A Type Thermostatic Water-circulator Bath: Liaoyang Boda Scientific Instrument Co., Ltd.
RM-6240 Polygrapher Recorder: Chengdu Instrument Co., Ltd.
FA1004 Electronic Analysis Balance: Shanghai Jingke Industrial Co., Ltd.

**Experimental Methods and Results:**

The antagonistic effects of the compounds of the present invention on the trachea constriction induced by histamine.
Guinea pigs were sacrificed and cut through the skin and subcutaneous tissue on ventral side of neck. Trachea was removed and cut from thyroid cartilage to trachea crotch, then placed into an ice cooled oxygenated Kebs-Hensleits solution. The connective tissues around the trachea were cut off. The trachea was held by a nipper on one end and cut into trachea strips (2cm×3mm) in helix. The samples were then put into a bath containing 20 ml of Kebs-Hensleits

solution. The lower end of the trachea strips was fastened to a vent hook and the upper end was connected to a strain gauge transducer, thus the tension change was measured by the recorder. The bath was controlled at 37°C and oxygen gas was continuously supplied. The preload of the samples was 2 g. The samples were equilibrated in the nutrient solution for 1 hour and the solution was refreshed every 20 min.

When the tension of the samples reached a stable value, various concentrations ($1x10^{-10}$~$3\times10^{-4}$ $mol \cdot L^{-1}$) of histamine were added to the bath. A dose-effect curve was established to determine the concentration of histamine when 50% of the maximum contraction was reached. After refreshing the solution and balancing the samples for 60 min, an amount of histamine was added to the bath. When the tension of trachea strips reached 50 % of the maximum contraction, the tested compounds were added to bath, and the antagonistic effect (represented as relaxing rate) of the compounds on the isolated trachea constriction induced by histamine was calculated as below:

$$\text{Relaxing rate (\%)} = [(\text{Contraction intensity after histamine addition} - \text{Contraction intensity after tested compounds addition})/\text{Contraction intensity after tested compounds addition}] \times 100\%$$

The relaxing rate of each compound was presented in Table 1.

Table 1 Antagonistic effect of the active compounds on trachea contraction induced by histamine

| Structure of the compounds | Relaxing rate (%) | Structure of the compounds | Relaxing rate (%) |
|---|---|---|---|
| 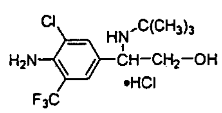 | 123.5 | (+) 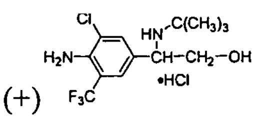 | 102.5 |
| (-) 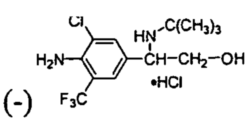 | 139.5 | | |
| 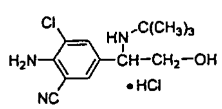 | 100.0 | (+) 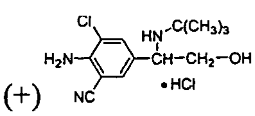 | 78.1 |
| (-) 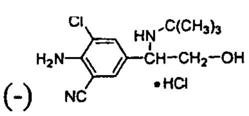 | 112.1 | | |
| 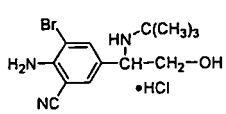 | 73.1 | (+) 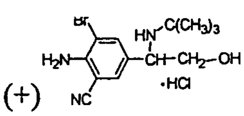 | 61.9 |
| (-) 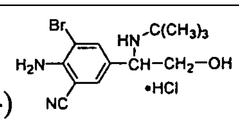 | 85.3 | | |
| 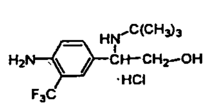 | 70.2 | (+) 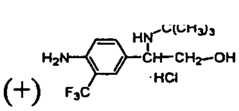 | 55.3 |

(continued)

| Structure of the compounds | Relaxing rate (%) | Structure of the compounds | Relaxing rate (%) |
|---|---|---|---|
| (-) 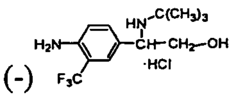 | 88.3 | | |

## Claims

**1.** Compounds of formula (1) having (-) or (+) configuration,

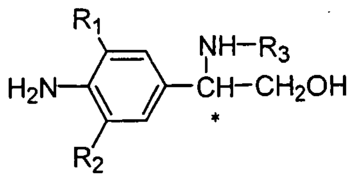

(I)

wherein

$R_1$ is H or halo; $R_2$ is $CF_3$, CN, or halo; $R_3$ is linear or branched alkyl having 1 to 6 carbon atoms, or cycloalkyl having 3 to 6 carbon atoms,

or pharmaceutically acceptable salts thereof.

**2.** The compound according to Claim 1, wherein $R_1$ is Cl or Br.

**3.** The compound according to Claim 1, wherein $R_2$ is $CF_3$, CN, or F.

**4.** The compound according to Claim 1, wherein $R_3$ is linear or branched alkyl having 3 to 6 carbon atoms.

**5.** The compound according to Claim 1, wherein the compounds of formula (I) have (-) configuration.

**6.** The compound according to Claim 1, selected from the group consisting of:

(-)-2-(3-chloro-4-amino-5-trifluoromethylphenyl)-2-tert-butylamino-ethanol hydrochloride,
(+)-2-(3-chloro-4-amino-5-trifluoromethylphenyl)-2-tert-butylamino-ethanol hydrochloride,
(-)-2-(3-trifluoromethyl-4-aminophenyl)-2-tert-butylamino-ethanol hydrochloride,
(+)-2-(3-trifluoromethyl-4-aminophenyl)-2-tert-butylamino-ethanol hydrochloride,
(-)-2-(3-chloro-4-amino-5-cyanophenyl)-2-tert-butylamino-ethanol hydrochloride,
(+)-2-(3-chloro-4-amino-5-cyanophenyl)-2-tert-butylamino-ethanol hydrochloride,
(-)-2-(3-bromo-4-amino-5-cyanophenyl)-2-tert-butylamino-ethanol hydrochloride, and
(+)-2-(3-bromo-4-amino-5-cyanophenyl)-2-tert-butylamino-ethanol hydrochloride.

**7.** A method for preparing the compounds of Claim 1, comprising reacting the racemic mixture of formula (II)

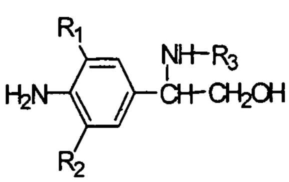

(II)

wherein $R_1$, $R_2$ and $R_3$ are as defined in Claim 1,

with a compound selected from the group consisting of: D(-)-tartaric acid, L(+)-tartaric acid, dibenzoyl-D-tartaric acid, dibenzoyl-L-tartaric acid, (+)(-)camphor-10-sulfonic acid, L(-)-malic acid, L(+)-mandelic acid, d-α-bromo-camphorsulfonic acid and 1-quininic acid, under an anhydrous condition to form a salt; and
crystallizing the salt for two or more times to resolve it into compounds of formula (I) of Claim 1 having optical activity.

**8.** A pharmaceutical composition comprising the compounds according to any one of Claims 1-6 and pharmaceutically acceptable excipients.

**9.** Use of the compound according to any one of Claims 1-6 in the preparation of medicaments having effect of $\beta_2$-receptor agonist.

**10.** Use of the compound according to any one of Claims 1-6 in the preparation of medicaments for the treatment of asthma or bronchitis.

**Amended claims under Art. 19.1 PCT**

**1.** Compounds of formula (I) having (-) configuration,

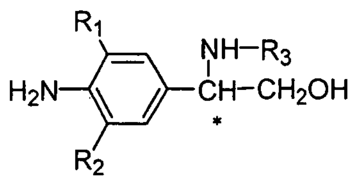

(I)

wherein
$R_1$ is H or halo; $R_2$ is $CF_3$, CN, or halo; $R_3$ is linear or branched alkyl having I to 6 carbon atoms, or cycloalkyl having 3 to 6 carbon atoms,
or pharmaceutically acceptable salts thereof.

**2.** The compound according to Claim 1, wherein $R_1$ is Cl or Br.

**3.** The compound according to Claim 1, wherein $R_2$ is $CF_3$, CN, or F.

**4.** The compound according to Claim 1, wherein $R_3$ is linear or branched alkyl having 3 to 6 carbon atoms.

**5.** The compound according to Claim 1, wherein the compounds of formula (I) have (-) configuration.

**6.** The compound according to Claim 1, selected from the group consisting of:

(-)-2-(3-chloro-4-amino-5-trifluoromethylphenyl)-2-tert-butylamino-ethanol hydrochloride,
(-)-2-(3-trifluoromethyl-4-aminophenyl)-2-tert-butylamino-ethanol hydrochloride,
(-)-2-(3-chloro-4-amino-5-cyanophenyl)-2-tert-butylamino-ethanol hydrochloride, and
(-)-2-(3-bromo-4-amino-5-cyanophenyl)-2-tert-butylamino-ethanol hydrochloride.

**7.** A method for preparing the compounds of Claim 1, comprising
reacting the racemic mixture of formula (II)

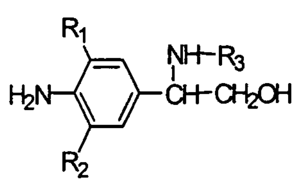

(II)

wherein $R_1$, $R_2$ and $R_3$ are as defined in Claim 1,
with a compound selected from the group consisting of: D(-)-tartaric acid, L(+)-tartaric acid, dibenzoyl-D-tartaric acid, dibenzoyl-L-tartaric acid, (+)(-)camphor-10-sulfonic acid, L(-)-malic acid, L(+)-mandelic acid, d-α-bromo-camphorsulfonic acid and 1-quininic acid, under an anhydrous condition to form a salt; and
crystallizing the salt for two or more times to resolve it into compounds of formula (I) of Claim 1 having optical activity.

**8.** A pharmaceutical composition comprising the compounds according to any one of Claims 1-6 and pharmaceutically acceptable excipients.

**9.** Use of the compound according to any one of Claims 1-6 in the preparation of medicaments having effect of $\beta_2$-receptor agonist.

**10.** Use of the compound according to any one of Claims 1-6 in the preparation of medicaments for the treatment of asthma.

# INTERNATIONAL SEARCH REPORT

International application No.
PCT/CN2007/002060

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC C07C A61K A61P

Documentation searched other than minimum documentation to the extent that such documents are included in the fields searched

Electronic data base consulted during the international search (name of data base and, where practicable, search terms used)

CNPAT, CNKI, EPODOC, WPI, PAJ, STN: phenylethanolamine?, resolut+, asthma, bronchitis, butylamino ethanol

C. DOCUMENTS CONSIDERED TO BE RELEVANT

| Category* | Citation of document, with indication, where appropriate, of the relevant passages | Relevant to claim No. |
|---|---|---|
| Y | CN1408703A (SHENYANG PHARMACEUTICAL UNIVERSITY ) 09 Apr. 2003(09.04.2003) claims, examples | 1－10 |
| Y | CN1273966A (CHENGDU ORGANIC CHEM INST CAS) 22 Nov. 2000 (22.11.2000) claims, page 1 paragraph 4 of description | 1－10 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 17 Sep. 2007 (17.09.2007) | **11 Oct. 2007 (11.10.2007)** |

Name and mailing address of the ISA/CN
The State Intellectual Property Office, the P.R.China
6 Xitucheng Rd., Jimen Bridge, Haidian District, Beijing, China
100088
Facsimile No. 86-10-62019451

Authorized officer
LV Qing
Telephone No. (86-10)62085607

Form PCT/ISA /210 (second sheet) (April 2007)

**INTERNATIONAL SEARCH REPORT**
Information on patent family members

International application No.
PCT/CN2007/002060

| Patent Documents referred in the Report | Publication Date | Patent Family | Publication Date |
|---|---|---|---|
| CN1408703A | 09.04.2003 | WO03093219A1 | 13.11.2003 |
| | | AU2002338124A1 | 17.11.2003 |
| | | EP1439164A1 | 21.07.2004 |
| | | US2004266867A1 | 30.12.2004 |
| | | JP2005519989T | 07.07.2005 |
| | | AU2002338124A8 | 17.11.2003 |
| | | RU2264382C1 | 20.11.2005 |
| | | US7098364B2 | 29.08.2006 |
| | | CN1276911C | 27.09.2006 |
| | | EP1439164B1 | 11.04.2007 |
| | | DE60219500E | 24.05.2007 |
| CN1273966A | 22.11.2000 | CN1173929C | 03.11.2004 |

Form PCT/ISA /210 (patent family annex) (April 2007)

## INTERNATIONAL SEARCH REPORT

International application No.
PCT/CN2007/002060

Continuation of: CLASSIFICATION OF SUBJECT MATTER

C07C215/28(2006.01)i
C07C213/10(2006.01)n
A61K 31/137(2006.01)n
A61P11/06(2006.01)n
A61P11/08(2006.01)n

Form PCT/ISA /210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 01128234